Europäisches Patentamt

European Patent Office

Office européen des brevets

(19)

(11)  EP 0 885 061 B1

(12)  FASCICULE DE BREVET EUROPEEN

(45) Date de publication et mention
de la délivrance du brevet:
20.02.2002  Bulletin 2002/08

(51) Int Cl.⁷: **B01J 13/02**, A61K 9/50,
A61K 7/40

(21) Numéro de dépôt: 97947105.9

(22) Date de dépôt: 20.11.1997

(86) Numéro de dépôt international:
PCT/FR97/02095

(87) Numéro de publication internationale:
WO 98/22210 (28.05.1998 Gazette 1998/21)

(54) **MICROCAPSULES DE CHITINE OU DE DERIVES DE CHITINE CONTENANT UNE SUBSTANCE HYDROPHOBE, NOTAMMENT UN FILTRE SOLAIRE ET PROCEDE DE PREPARATION DE TELLES MICROCAPSULES**

MIKROKABELN AUS CHITIN ODER CHITINDERIVATEN, DIE EINE HYDROPHOBE SUBSTANZ, INSBESONDERE EIN SONNENSCHUTZFILTER ENTHALTEN, UND METHODE ZUR HERSTELLUNG SOLCHER MIKROKAPSELN

CHITIN OR CHITIN DERIVATIVE MICROCAPSULES CONTAINING A HYDROPHOBIC SUBSTANCE, PARTICULARLY A SUN FILTER AND METHOD FOR PREPARING SUCH MICROCAPSULES

(84) Etats contractants désignés:
**AT BE CH DE DK ES FI FR GB GR IE IT LI LU MC NL PT SE**

(30) Priorité:  **21.11.1996  FR 9614215**

(43) Date de publication de la demande:
**23.12.1998   Bulletin 1998/52**

(73) Titulaire: **MERCK S.A.**
**94120 Fontenay-sous-Bois (FR)**

(72) Inventeurs:
• **GRANDMONTAGNE, Bernard**
**F-29880 Plouguerneau (FR)**
• **MARCHIO, François**
**F-75020 Paris (FR)**

(74) Mandataire: **Warcoin, Jacques**
**Cabinet Régimbeau 20, rue de Chazelles**
**75847 Paris cedex 17 (FR)**

(56) Documents cités:
**EP-A- 0 342 557**      **EP-A- 0 615 979**

**Description**

La présente invention concerne le domaine des microcapsules contenant une substance hydrophobe (ou liposoluble) d'intérêt.

Elle concerne également un procédé de préparation de ces microcapsules et l'application de celles-ci en cosmétique, pharmacie notamment.

L'usage des émulsions, eau dans huile, huile dans eau ou de variantes de ces dernières, sont des solutions intéressantes lorsque l'on souhaite apporter des composés actifs liposolubles au contact de la peau. Cette technique très utilisée dans les soins cosmétiques et pharmaceutiques, atteint ses limites lorsque, par exemple, la molécule ne doit ni diffuser dans les couches cornées, ni s'évaporer, ni s'éliminer de toute autre manière.

L'usage de microcapsules ou nanocapsules (définies ci-après par le terme générique "microcapsules") contenant des dérivés liposolubles est une alternative intéressante aux émulsions en particulier lorsque l'on souhaite faire durer le contact d'une molécule avec le derme. Bien maîtrisées, les capsules peuvent emprisonner des actifs, les maintenir isolés ou les libérer à volonté.

En effet si pour certains dérivés, il est impératif de les maintenir concentrés afin d'en préserver l'efficacité, voire de l'accroître, pour d'autres leur diffusion dans le temps est souhaitable. L'usage de molécules encapsulées permet l'un ou l'autre.

En particulier, dans le domaine de la protection solaire, les altérations et les vieillissements cutanés accélérés dus aux UVA et UVB ont sensibilisé les populations aux dangers du rayonnement solaire et accentué le besoin en crèmes protectrices. Les éléments actifs principaux de ces cosmétiques sont des filtres solaires qui quoique très efficaces ne sont pas toujours très bien tolérés par toutes les peaux.

Afin de limiter, voire de supprimer cette difficulté, il apparaît souhaitable de diminuer le contact avec la peau lors de l'application de ces produits en emprisonnant les filtres dans des capsules.

On a par exemple proposé dans la demande de brevet EP-A-0 509 904 des microcapsules contenant un filtre solaire obtenues par coacervation complexe au moyen de deux solutions aqueuses colloïdales contenant des polymères de charge électrique opposée.

La. coacervation complexe est généralement appliquée à l'encapsulation d'un matériau de caractère lipophile émulsionné dans une solution aqueuse de polymères destinés à former la paroi des microcapsules.

On sait en effet de façon générale que lorsque deux solutions aqueuses colloïdales contenant respectivement un polymère anionique et un polymère cationique sont mélangées en présence d'un liquide en émulsion ou d'un solide en suspension, il se forme un coacervat déposé autour des noyaux liquides ou solides pour former une paroi liquide les isolant du milieu. Le durcissement in situ de cette paroi liquide par un réticulant approprié à la nature des polymères permet d'obtenir des microparticules stables en suspension.

Ces microparticules sont généralement formées à base de gélatine en tant que polymère cationique et de polysaccharide en tant que polymère anionique.

Il est généralement possible de réaliser par coacervation des capsules de tailles variables et voisines de 10 à 100 μm.

Ces systèmes ne sont pas totalement satisfaisants en raison soit du type de polymères utilisés pour réaliser la membrane, comme le collagène, les gélatines, les guars, ou les gommes arabiques, seuls ou en mélange, soit, en raison des tailles obtenues, lors de l'utilisation de polysaccharides par exemple.

En effet, pour que la protection soit efficiente et n'engendre plus d'intolérances cutanées, les capsules doivent être particulièrement imperméables. De plus, pour que l'efficacité photochimique perdure, à concentration égale, les filtres doivent être les plus dispersés possible et ne pouvant être diluées, les capsules les plus petites possibles.

La taille est, en outre, un facteur essentiel de l'efficacité du filtre. Plus la taille est faible plus la protection croit.

Il est également possible de réaliser des systèmes matriciels pleins, de très petites tailles, inférieurs au micron, à partir de cires préalablement fondues, émulsionnées et refroidies. Néanmoins ces techniques drastiques donnent des sphères perméables, instables dans les formulations cosmétiques traditionnelles. Leur faible durée de vie en limite l'intérêt.

Une telle réalisation est décrite par exemple dans la demande de brevet WO-A-95/28912.

On a maintenant trouvé de manière inattendue qu'il était possible de réaliser des capsules de chitine ou d'un de ses dérivés, de taille inférieure au μm, répondant à la fois aux exigences d'imperméabilité et de très petite taille et dont la biocompatibilité organique en assure l'innocuité.

La chitine est le polymère de structure des arthropodes, crustacés et insectes et fait partie de la membrane de certains champignons. Elle assure la tenue de l'endosquelette des céphalopodes. Elle est au monde animal le pendant de ce qu'est la cellulose au règne végétal.

Sa particularité réside dans son inertie chimique. Peu réactive, insoluble dans la plupart des solvants organiques connus, elle ne peut, non plus être thermoformée. Ce n'est qu'en la transformant en chitosane qu'il devient possible de la mettre en oeuvre.

Pour moduler la chitine, il est nécessaire de procéder en deux étapes. La première consiste à transformer la chitine en chitosane puis la seconde à retransformer le chitosane en chitine.

Il est ainsi décrit dans la demande de brevet EP-A-0 013 181, page 2, une préparation de chitine par N-acétylation de chitosane avec de l'anhydride acétique dans une solution de pyridine ou une solution d'acide perchlorique.

Des dérivés de chitine ont également été préparés. La demande de brevet. EP-A-0 013 181 décrit la préparation de N-acétyle carboxyalkylchitine par acétylation de carboxyalkylchitine déacétylée avec un anhydride d'un acide organique. D'autres dérivés de chitine sont également mentionnés tels que les hydroxyalkychitines. Le matériau obtenu peut être mis sous la forme de particules sphériques utilisables comme résines échangeuses d'ions.

La demande de brevet EP-A-0 021 750 décrit un procédé de préparation de chitine par acylation de chitine déacétylée en présence d'un anhydride d'acide organique et d'un agent de suspension tel qu'un monoester de sorbitan.

La demande de brevet EP-A-0 026 618 décrit un matériau comprenant un mélange de deux ou plus dérivés de chitine éthérifiés obtenus par le même procédé d'acétylation indiqué pour les deux documents précités. Les matériaux sphériques obtenus peuvent être notamment utilisés comme résines échangeuses d'ions.

Cependant, ces documents ne décrivent en aucune façon des microcapsules de chitine ou de dérivés de chitine.

De manière inattendue, les inventeurs ont mis en évidence qu'il était possible de transformer de façon stable un sel alkylsulfaté ou arylsulfaté ou phosphaté de chitosane ou de dérivés de chitosane par acétylation ou par réticulation ou toute autre méthode de condensation. De plus, cette transformation stable peut également être appliquée aux autres polymères qui réagissent dans les mêmes conditions que les sels de chitosane précités, à savoir les polyamines polyhydroxylées de synthèse. Ainsi, l'invention s'étend également aux polyamines polyhydroxylées de synthèse se condensant en présence de tensio-actifs sulfatés ou phosphatés pour donner après acétylation ou réticulation des sels stables.

La présente invention est relative à de nouvelles microcapsules caractérisées en ce qu'elles sont formées d'une paroi de chitine ou de dérivés de chitine ou de polyamines polyhydroxylées ou un sel de ceux-ci, ladite paroi enrobant une substance hydrophobe.

La présente invention est plus particulièrement relative à de nouvelles microcapsules caractérisées en ce qu'elles sont formées d'une paroi de chitine ou de dérivés de chitine ou un sel de ceux-ci, ladite paroi enrobant une substance hydrophobe.

Ces microcapsules présentent, outre leur imperméabilité contrôlée et leur biocompatibilité, plusieurs autres propriétés physiques remarquables dont on peut citer :

- insolubilité dans tous les solvants cosmétiques usuels et solvants organiques,
- étanchéité dans les milieux cosmétiques,
- dispersibilité élevée en milieu aqueux ou hydroalcoolique,
- un potentiel zeta positif
- capacité à se disperser sur la surface de la peau de manière homogène et substantive du fait de leur charge cationique résiduelle.

Par microcapsule, on entend toute particule formée d'une paroi enrobant une substance d'intérêt hydrophobe.

On distingue de ce fait les microcapsules des microsphères dont le. matériau polymérique forme matrice et dans lequel est noyée (en anglais "embedded") ladite substance.

Par microcapsule, on entend aussi bien les microcapsules strictes que les nanocapsules (de taille moyenne inférieure au micromètre).

Par ailleurs, le terme paroi de microparticules signifie que cette paroi est stable à la différence d'un coacervat. Le terme "paroi" fait donc référence à une stabilisation par acétylation ou réticulation.

On notera que le document EP-A-615 979 décrit des carboxyméthyl polysaccharides, notamment la carboxyméthylchitine, partiellement estérifiés. Mais, ce document ne décrit ni ne suggère en aucune manière des microcapsules de chitine. Il ne suffit pas en effet de noter à la colonne 3, lignes 16 et 17 que ces esters peuvent être utiles en galénique pour la préparation de capsules ou de microcapsules pour permettre à l'homme du métier de réaliser ces microcapsules alors que la difficulté réside justement dans cette réalisation.

De même le document EP-A-342557 décrit les mêmes esters de carboxyméthylchitine et se réfère simplement, colonne 3, lignes 26 et 27, à l'utilisation de ces esters pour la préparation de microcapsules. Ce document ne décrit ni ne suggère non plus la préparation de ces microcapsules ainsi que les microcapsules obtenues.

Par ailleurs, il existe une différence essentielle entre le carboxyméthylchitine et la chitine ou dérivés de chitine selon l'invention.

La carboxyméthylchitine est du type R-O-CH2-CO2$\ominus$. Il s'agit de dérivés solubles.

Par contre, les dérivés de chitine selon l'invention qui ont donc des propriétés similaires à la chitine sont bien évidemment insolubles. On rappelle qu'il s'agit de constituants principaux des carapaces des crustacés qui vivent dans l'eau.

L'invention concerne particulièrement les microcapsules formées d'une paroi de chitine.

La chitine ou poly N-acétyl-D-glucosamine est un polysaccharide dont les unités sont liées entre elles par des ponts β-1,4 glycosidiques. Elle se distingue donc du chitosane qui est une chitine N-désacétylée. Lorsque la désacétylation est totale, on obtient un chitosane singulier, le polyglucosamine.

La chitine est naturellement désacétylé au-dessus de 5% et plus. Le terme de chitosane est réservé aux dérivés qui, ayant subi une désacétylation, deviennent soluble en milieu aqueux acide. Il faut pour ce faire un degré de désacétylation au moins supérieur à 65%. Ce taux influera sur les propriétés physiques du chitosane, solubilité et viscosité, mais également sur son comportement biochimique.

La chitine, le chitosane, leurs oligomères et monomères ne sont pas immunogènes. Ils sont biocompatibles et très bien tolérés par les tissus vivants. La LD 50 du chitosane est de 18 g par kg (valeur très proche de celle du sucre de table). Ces caractéristiques en font un produit très facilement contaminable et il n'est pas rare d'y trouver de 500 000 à 1 million de germes au g de matière brute.

Les techniques de stérilisation des produits sont aujourd'hui bien connues. L'irradiation Gamma peut être employée lorsque l'on cherche une stérilisation totale. Sinon de nombreux conservateurs permettent de maintenir des solutions en dessous de 1000 germes/g (contraintes cosmétiques). La stérilisation autoclave peut être réalisée sur les produits bruts, sur les gels, mais doit être évitée sur les solutions qui, elles, se dégradent rapidement au-dessus de 40°C.

Les dérivés de chitine concernés par l'invention sont notamment les dérivés éthérifiés de chitine tels que ceux décrits par les demandes de brevet EP-A-0 013 181, EP-A-0 021 750, EP-A-0 026 618 précitées.

En particulier, les dérivés O-carboxyalkyle, O-hydroxyalkyle, O-dihydroxyalkyle, O-alkyle. On citera également les dérivés de chitine comprenant les dérivés N-substitués, notamment N-carboxyalkyle, N-hydroxyalkyle, N-dihydroxyalkyle, N-alkyle.

Les dérivés de chitine ou les polyamines polyhydroxylées constituant la paroi peuvent également résulter de la réticulation du chitosane ou d'un dérivé de chitosane ou des polyamines polyhydroxylées par un agent tel que le glutaraldéhyde ou un diisocyanate.

Les sels de chitine ou des dérivés de chitine ou les polyamines polyhydroxylées sont notamment les sels de métal alcalin ou alcalino terreux, d'ammonium, d'acide acétique, d'acide chlorhydrique, etc.

De préférence, l'invention concerne des microcapsules de chitine.

Les microcapsules comprennent en général 5 à 50 % en poids de substance hydrophobe, de préférence entre 33 et 45 %.

Elles présentent avantageusement une taille moyenne comprise entre 0,1 μm et 30 μm, de préférence une taille moyenne comprise entre 0,3 et 10 μm.

Elles présentent de préférence un potentiel zeta compris entre 20 mV et 50 mV.

La substance hydrophobe peut être un composé solide à la température d'utilisation des microcapsules mais, de préférence, une substance liquide qui peut être le composé d'intérêt lui-même ou le composé d'intérêt en solution dans un solvant organique.

Ces substances sont notamment choisies parmi les composés à usage cosmétique, pharmaceutique, les agents chromogènes, éventuellement en solution organique. De préférence, la substance hydrophobe est un filtre solaire pur, ou dilué dans une huile, choisi dans le groupe suivant :

esters cinnamiques, acide et esters para-amino benzoïques, esters salicyliques, benzylidène camphre et dérivés, benzophénones, dibenzoylméthanes, benzyl diphényl acrylates, anthranilates, triazines, et plus précisemment : octyl méthoxycinnamate, octyl diméthyl PABA, octyl salicylate, homomenthyl salicylate, 4-méthylbenzilidène camphre, benzophénone 3, butyl méthoxy-dibenzoylméthane, octocrylène, menthyl anthranilate, triazone...

De préférence, les composés sont choisis dans le groupe suivant : 2-éthylhexyl-p-méthoxycinnamate, 2-éthylhexyl p-diméthylaminobenzoate, 2-éthylhexyl salicylate, 2-éthylhexyl-2-cyano-3,3-diphényl acrylate, 4-méthylbenzylidène camphre, 2-hydroxy-4-méthoxy benzophénone, 4-4-butyl-4'-methoxydibenzoylméthane.

Cette liste n'est pas limitative et l'invention s'étend à d'autres filtres solaires bien connus dans l'art considéré.

L'invention concerne également une composition à usagé cosmétique ou pharmaceutique pour une application topique externe comprenant une quantité efficace de microcapsules selon l'invention contenant une substance d'intérêt cosmétique ou pharmaceutique et un véhicule inerte.

Notamment, l'invention concerne une composition cosmétique ou pharmaceutique pour un usage topique externe absorbant les rayonnements UV en particulier UVB et UVA comprenant les microcapsules selon l'invention contenant un filtre solaire, en dispersion dans un véhicule inerte.

Ces compositions peuvent se présenter sous la forme par exemple de gels, crèmes et huiles.

L'invention concerne également un procédé pour protéger la peau contre les rayonnements UV, notamment UVA ou UVB, caractérisé en ce que l'on applique sur la peau une quantité efficace d'une composition cosmétique précitée.

L'invention concerne également une composition comprenant des microcapsules selon l'invention contenant un agent chromogène.

L'invention concerne également un procédé de préparation des microcapsules selon l'invention. Le procédé est caractérisé en ce que :

- on forme une émulsion d'une phase hydrophobe, constituée de la substance hydrophobe, dans une phase aqueuse contenant un agent tensio-actif anionique choisi parmi des composés susceptibles de provoquer une insolubilisation de chitosane ou de dérivés de chitosane ou de polyamines polyhydroxylées,
- on mélange ladite émulsion avec un sel organique de chitosane ou de dérivés de chitosane ou de polyamines polyhydroxylées de façon à provoquer la coacervation du chitosane ou des polyamines polyhydroxylées ou des dérivés de chitosane autour des gouttelettes de phase hydrophobe,
- on soumet le coacervat de chitosane ou de polyamines polyhydroxylées ou de dérivés de chitosane à une réaction d'acétylation ou de réticulation,
- on récupère les microcapsules.

Le procédé d'encapsulation selon l'invention comprend deux étapes. La première aboutit à des capsules à membrane de chitosane ou dérivés de chitosane ou de polyamines polyhydroxylées puis par acétylation ou réticulation, ce polymère est transformé en chitine ou dérivés de chitine ou de polyamines polyhydroxylées acétylés ou réticulés, afin d'en accroître la stabilité.

La première étape est elle même divisée en deux parties dont la mise en oeuvre peut-être simultanée ou non. La première consiste à réaliser une émulsion, c'est-à-dire une suspension de gouttelettes lipidiques dans une phase aqueuse, la seconde à enrober ces gouttelettes d'une membrane de chitosane ou de dérivés de chitosane ou de polyamines polyhydroxylées.

Pour ce faire, on mélange une substance hydrophobe, un ou plusieurs filtres solaires ou encore des solutions de filtres en milieu organique liposoluble, à un tensio-actif en solution aqueuse choisi parmi les tensio-actifs susceptibles de provoquer une condensation de chitosane ou de dérivés de chitosane ou de polyamines polyhydroxylées. Parmi les tensio-actifs on emploie avantageusement les dérivés sulfatés, phosphonatés ou phosphatés alcalins ou alcalino-terreux, de préférence les dérivés sulfatés.

Parmi ces dérivés, on choisit avantageusement les dérivés présentant un reste alkyle en $C_6$-$C_{18}$, aryle en $C_6$-$C_{10}$, alkyle ($C_1$-$C_{10}$) aryle ($C_6$) ou aryle ($C_6$) alkyle ($C_1$-$C_{10}$). De préférence on choisit un alkyle ($C_6$-$C_{18}$) sulfate sous sa forme alcaline notamment sodée et de préférence encore le lauryl sulfate sodé.

Ce tensio-actif est initialement saturé dans le mélange. Il peut y être ajouté, tout composé facilitant le mélange ou accentuant les propriétés tensio-actives.

Une meilleure efficacité (taille des particules) peut être obtenue par addition d'un ou de plusieurs tensio-actifs supplémentaires correctement choisis parmi les composés connus pour cette action. Divers fluides silicones hydrosolubles améliorent notablement le procédé.

Ce mélange est ensuite rajouté sous forte agitation à une solution de chitosane ou dérivés de chitosane ou de polyamines polyhydroxylées préalablement ou simultanément réalisée.

Le chitosane ou les dérivés de chitosane ou les polyamines polyhydroxylées se condensent à la surface des vésicules lipidiques en un sel sulfaté de chitosane ou dérivés de chitosane ou de polyamines polyhydroxylées insolubles. Chaque nouveau mouvement d'agitation provoque irréversiblement la scission d'un miscelle en deux nouvelles vésicules. La taille des vésicules décroît rapidement, l'addition de tensio-actifs accentue le mécanisme.

Cette technique aboutit après quelques minutes d'agitation à l'obtention d'une solution aqueuse contenant des capsules de taille voisine par puissance de dix au micron, caractérisées par une membrane constituée d'un sel de tensio-actif (notamment alkylsulfate) de chitosane ou dérivés de chitosane ou de polyamines polyhydroxylées.

Dans l'eau, la solvatation des chitosanes ou dérivés de chitosane ou de polyamines polyhydroxylées salifiés est assurée à un pH inférieur à environ 6,5. Le sel utilisé est généralement un acétate mais d'autres ions organiques peuvent également être utilisés, notamment ceux issus de la dissociation des monoacides faibles organiques de type alkyle ou aryle ou hydroxy alkylcarboxyliques et dérivés acides, tels lactique, glutamique, gluconique, glycolique, benzoïque, aminobenzoïque, etc, ou d'ions dont l'équilibre salin avec le chitosane peut être déplacé par réaction d'acétylation (ce qui exclut les acides forts de type chlorhydrique, sulfurique, phosphorique et nitrique).

Les dérivés de chitosane pouvant être utilisés sont notamment les O-ou N- carboxyalkylchitosane mais peuvent être également les O- ou N-hydroxyalkyle ou alkylchitosanes.

Le chitosane est un polymère soluble en milieu aqueux acide. La chitine est un polymère insoluble particulièrement imperméable. Afin de rendre irréversible l'emprisonnement des huiles dans les capsules et d'en limiter leur destruction ou leur évolution par déplacement de sels acido-basiques, on renforce la membrane en transformant le chitosane par acétylation ou réticulation.

La réaction d'acétylation est assurée par un anhydride d'acide organique, notamment un acide organique aliphatique, de 4 à 40 atomes de carbone, aromatique de 12 à 20 atomes de carbone tels que l'anhydride acétique, l'anhydride propionique, l'anhydride butyrique, l'anhydride valérique, l'anhydride benzoïque.

Ces anhydrides sont mis en solution dans un solvant polaire permettant la réaction d'acétylation, notamment l'alcool éthylique, le tetrahydrofuranne, le dioxane etc.

La réaction d'acétylation est bien connue en soi.

L'addition d'anhydride déplace l'équilibre ionique acido basique unissant le tensio-actif et l'amine du chitosane ou de l'un de ses dérivés ou les polyamines polyhydroxylées et permet de réaliser ainsi l'acétylation.

D'autres variantes consistent à réticuler le coacervat de chitosane ou dérivés de chitosane ou les polyamines polyhydroxylées par réticulation interfaciale au moyen de glutaraldéhyde ou de diisocyanates. Cette réaction est bien connue en microencapsulation.

L'irréversibilité du procédé peut également être obtenue par mise en oeuvre d'interactions polyanions-polycations par le biais de l'action d'un polymère anionique tel un carbopol ou un alginate ou encore d'un polymère sulfaté tel un carraghénane ou un fucoïdane.

Selon une variante préférée du procédé prise en combinaison avec les précédentes, les quantités pondérales des différents réactifs utilisés sont les suivantes :

- substance hydrophobe        5 % à 50 % de préférence 33 à 45 %
- tensio-actifs        0,1 % à 1,5 % de préférence environ 0,33 %
- sel de chitosane ou de dérivés de chitosane ou de polyamines polyhydroxylées        0,1 % à 10 % de préférence environ 2 %
- anhydride ou        0,1 % à 10 % de préférence environ 0,33 %
- réticulant        0,1 % à 10 % de préférence environ 0,33 %

Le procédé est également remarquable en ce qu'il peut être effectué à température ambiante sans apport d'énergie.

L'invention va maintenant être décrite en regard des exemples présentés ci-après destinés à illustrer l'invention.

Le procédé de préparation général décrit dans les exemples suivants consiste à mélanger deux phases sous agitation. La taille des particules obtenue est dépendante des vitesses d'agitation et donc du matériel employé. A titre d'exemple, une agitation réalisée par un simple arbre équipé d'une palle perpendiculaire et toumant à 8000 t/mn permet d'obtenir des capsules de taille inférieure à 10 μm. Si l'agitation est réalisée au turbomalaxeur à 24 000 t/mn, la taille moyenne des capsules est alors nettement inférieure à 1 μm.

EXEMPLES

Exemple 1

40 g de filtre solaire 2-éthylhexyl-p-méthoxycinnamate (EUSOLEX® 2292) sont saturés par 0,4 g de lauryl sulfate de sodium et 0,4 g de fluide 190 (silicone). Le tout est fortement agité à température ambiante et mélangé à 80 g d'une solution aqueuse contenant 2% de sel de chitosane (acétate). L'agitation est maintenue jusqu'à obtention de vésicules de tailles inférieures à 10 μm. Sur cette dispersion miscellaire est rajoutée une solution alcoolique acétylante d'anhydride acétique. Le tout est ensuite lavé par toute méthode appropriée nécessaire à l'élimination des produits en excès.

Les caractéristiques des microcapsules sont évaluées selon quatre mesures :

Potentiel Zeta

Equipement : Malvern instrument
Type cellule : ZEMO 10 Ref. Beam Mode F(Ka) =1.5 (Smoluchowsky)
Voltage cellule = 143.0 V
Température = 25,4°C
μ = 0.682 cp2        pH = 6,59
Conductivité cellule : 0,09 mS        constante diflectrique : 79,0
Position cellule : 17,00%

Résultats : Potentiel Zeta = +32,61 mV

Mobilité électrophorétique moyenne = 6,659 microns.cm/V.s.

Résistance physique des particules

Méthodes : Traitement de l'échantillon aux micro-ondes

Conditions: jusqu'à ébullition de la suspension
Résultats : Pas de déphasage de la suspension
Pas d'éclatement des capsules noté en microscopie optique (grossissement x 400)
Stabilité granulométrique (env. 1 μm).

Résistance à l'extraction

Filtre encapsulé : Octyl Methoxy Cinnamate (OMC), Eusolex® 2292.
Conditions : Type dilutest (mise en suspension dans différentes solutions tensio-actives et émulsions).
Filtration sur filtres Anotop® 0.2 μm
Prélèvement du filtrat/ Dilution solvant Eau /Ethanol : 20:80
Mesure : spectrophotométrie d'absorption UV; modèle Perkin Elmer

| SOLVANTS D'EXTRACTION | % DE FILTRE RELARGUE | TAUX D'ENCAPSULATION |
|---|---|---|
| Eau | non détectable | 100 |
| Emulsion H/E* | non détectable | 100 |
| Solution aqueuse à 5% de Simulsol 59 | 5 | 95 |
| Solution aqueuse à 5% de Incroquat Béhényl TMS | non détectable | 100 |

* Emulsion H/E à 18% de filtre encapsulé : cf/ mesure de l'indice de protection in vitro.

Analyse granulométrique

Equipement : MALVERN Instrument
Gamme : 30HD
Longueur faisceau : 2,4 mm
Obscuration: 9,3%
Dispersant R.I-1.3300

Résultats :

Type de distribution : volume   Densité = 3,260 g/cub.cm

concentration = 0,0023%

Les différents diamètres indiqués ci-après expriment de manière exhaustive le profil granulométrique général d'après le spectre enregistré.

D(v.0,5) diamètre moyen mesuré à 50 % de la courbe des cumuls de volume,
D(v.0,1) diamètre moyen mesuré à 10 % de la courbe des cumuls de volume,
D(V.0,9) diamètre moyen mesuré à 90 % de la courbe des cumuls de volume,
D(3,4) diamètre auquel figure le plus grand volume de particules (point culminant de la courbe de Gauss),
D(3,2) diamètre moyen des particules exprimé en population.

D(v.0,5) =     2,00 μm

D(v.0,1) = 0,63 µm
D(V.0,9) = 5,39 µm
D(3,4) = 2,62 µm
D(3,2) = 1,32 µm

Mesure de l'indice de protection in vitro d'émulsions contenant du 2-éthyl-p-méthoxycinnamate (OMC) encapsulé et non encapsulé

L'émulsion huile dans eau a la composition suivante en pourcentage en poids :

| | |
|---|---|
| Stéarate de glycéryle/stéarate de PEG-100 | 6 |
| Isohexadécane | 6 |
| PPG-15 stéaryl éther | 3 |
| Acide stéarique | 4 |
| Squalane | 2 |
| Phényl triméthicone | 0,5 |
| Diméthicone | 0,5 |
| PEG-8/tocophérol/acide ascorbique/acide citrique | 0,01 |
| Propylène glycol | 2 |
| Phénoxy éthanol/esters de paba | 0,8 |
| Eau déminéralisée | qsp 100 |

à laquelle est ajoutée de l'OMC encapsulé ou non.

Les différents diamètres expriment de manière exhaustive le profil granulométrique général d'après le spectre enregistré. Ainsi, la principale valeur D(v.0,5) correspond au diamètre moyen des particules, mesuré à 50 % de la courbe des cumuls de volume. En d'autres termes, à la valeur indiquée, 50 % du volume des particules se trouve sous cette valeur, 50 % au-dessus.

D(v,0.1) = diamètre moyen de la population des plus petites particules mesurées à 10 % des cumuls de volume, etc.

D(3,4) exprime le diamètre auquel figure le plus grand volume de particules (point culminant de la courbe de Gauss).

Enfin, D(3,2) correspondant au diamètre moyen des particules exprimé en population et non plus en volume. Cette valeur n'est pas directement exprimée par les mesures, mais est calculée d'après une formule mathématique. Le volume d'une grosse particule étant bien plus important que celui d'une petite particule, il convient de tenir compte également du nombre afin de compléter le profil statistique de l'échantillon analysé.

Méthode Diffey

Matériel et méthode

La préparation antisolaire est appliquée sur un substrat de type bande Transpore® (3M Cie), à surface bosselée similaire à la topographie du stratum cornéum.

Le principe revient à mesurer la transmission spectrale des radiations U.V. à travers ce ruban Transpore® avec et sans la préparation antisolaire.

La source d'irradiation U.V. (Lampe Xenon) émet un spectre luminescent à distribution continue entre 290 et 400 nm. L'émission luminescente traverse l'optique en quartz. 10 mm d'un spectroradiomètre (Optronic model 742, Optronic Labs. Inc.) connecté sur ordinateur. La calibration des longueurs d'ondes est obtenue au moyen d'une lampe à vapeur de mercure basse pression (253,7 nm et 435,8 nm).

Les doses d'application de la préparation à tester sont de 2 mg/cm$^2$ de support.

Le facteur de protection monochromatique à une longueur d'onde est donné d'après le "signal enregistré au travers de la bande vierge sur le signal mesuré en présence du produit à tester". Pour chaque produit, la transmission est déterminée sur 3 bandes différentes, et un facteur de protection moyen [PF($\lambda$)] est calculé avec l'écart type [$\Delta$P($\lambda$)].

Le facteur de protection solaire est évalué à partir des mesures de transmission d'après la formule :

$$SPF = \sum_{290}^{400} = E(\lambda)/ \sum_{290}^{400} E(\lambda)\, \varepsilon(\lambda)/PF(\lambda)$$

où E($\lambda$) est l'irradiance de la lumière solaire terrestre sous conditions définies et $\varepsilon(\lambda)$ est l'efficacité relative des UVR à la longueur d'onde $\lambda$ induisant l'érythème actinique d'après le spectre de référence adopté par le C.I.R.

Résultats :

| Taux matière active | OMC encapsulé | OMC pur |
|---|---|---|
| 6% | 6,92 | 6,74 |
| 10% | 12,05 | 11,05 |

Exemple 2

40 g de filtre solaire selon l'exemple 1 sont saturés par 0,4 g de lauryl sulfate de sodium. Le tout est fortement agité à température ambiante et mélangé à 80 g d'une solution aqueuse contenant 2% de sel de chitosane. L'agitation est maintenue jusqu'à obtention de vésicules de tailles souhaitées. Sur cette dispersion miscellaire est rajoutée une solution alcoolique acétylante d'anhydride phtalique. Le tout est ensuite lavé par toute méthode appropriée nécessaire à l'élimination des produits en excès.

Exemple 3

40 g de filtre solaire selon l'exemple 1 sont saturés par 0,4 g de lauryl sulfate de sodium. Le tout est fortement agité à température ambiante et mélangé à 80 g d'une solution aqueuse contenant 2% de sel de chitosane. L'agitation est maintenue jusqu'à obtention de vésicules de tailles souhaitées. Sur cette dispersion miscellaire est rajoutée une solution alcoolique acétylante d'anhydride propionique. Le tout est ensuite lavé par toute méthode appropriée nécessaire à l'élimination des produits en excès.

Exemple 4

40 g de filtre solaire selon l'exemple 1 sont saturés par 0,4 g de lauryl sulfate de sodium. Le tout est fortement agité à température ambiante et mélangé à 80 g d'une solution aqueuse contenant 2% de sel de chitosane. L'agitation est maintenue jusqu'à obtention de vésicules de tailles souhaitées. Sur cette dispersion miscellaire est rajoutée une solution aqueuse contenant 0.1 g de glutaraldéhyde. Le tout est ensuite lavé par toute méthode appropriée nécessaire à l'élimination des produits en excès. La membrane de la capsule est ensuite neutralisée par une solution de 0,1 à 0,5 g d'acide paraaminobenzoïque.

Exemple 5

40 g de filtre solaire selon l'exemple 1 sont saturés par 0,4 g de lauryl sulfate de sodium. Le tout est fortement agité à température ambiante et mélangé à 80 g d'une solution aqueuse contenant 2% de sel de. chitosane. L'agitation est maintenue jusqu'à obtention de vésicules de tailles souhaitées. Sur cette dispersion miscellaire est rajoutée sous agitation, une solution d'alginate de sodium à 0,5% afin de créer un complexe polyanion polycation de condensation. Le tout est ensuite lavé par toute méthode appropriée nécessaire à l'élimination des produits en excès.

Exemple 6

40 g de filtre solaire 2-éthylhexyl p-diméthylaminobenzoate (EUSOLEX 6007®) sont saturés par 0,4 g de lauryl sulfate de sodium et 0,4 g de fluide 190 (silicone). Le tout est fortement agité à température ambiante et mélangé à 80 g d'une solution aqueuse contenant 2% de sel de chitosane. L'agitation est maintenue jusqu'à obtention de vésicules de tailles inférieures à 10 μm. Sur cette dispersion miscellaire est rajoutée une solution alcoolique acétylante d'anhydride acétique. Le tout est ensuite lavé par toute méthode appropriée nécessaire à l'élimination des produits en excès.

Exemple 7

40 g de filtre solaire précédent sont saturés par 0,4 g de lauryl sulfate de sodium. Le tout est fortement agité à température ambiante et mélangé à 80 g d'une solution aqueuse contenant 2% de sel de chitosane. L'agitation est maintenue jusqu'à obtention de vésicules de tailles souhaitées. Sur cette dispersion miscellaire est rajoutée une solution alcoolique acétylante d'anhydride phtalique. Le tout est ensuite lavé par toute méthode appropriée nécessaire à l'élimination des produits en excès.

Exemple 8

40 g de filtre solaire précédent sont saturés par 0,4 g de lauryl sulfate de sodium. Le tout est fortement agité à température ambiante et mélangé à 80 g d'une solution aqueuse contenant 2% de sel de chitosane. L'agitation est maintenue jusqu'à obtention de vésicules de tailles souhaitées. Sur cette dispersion miscellaire est rajoutée une solution aqueuse contenant 0,1 g de glutaraldéhyde. Le tout est ensuite lavé par toute. méthode appropriée nécessaire à l'élimination des produits en excès. La membrane de la capsule est ensuite neutralisée par une solution de 0,1 à 0,5 g d'acide paraaminobenzoïque.

Exemples 9 à 13

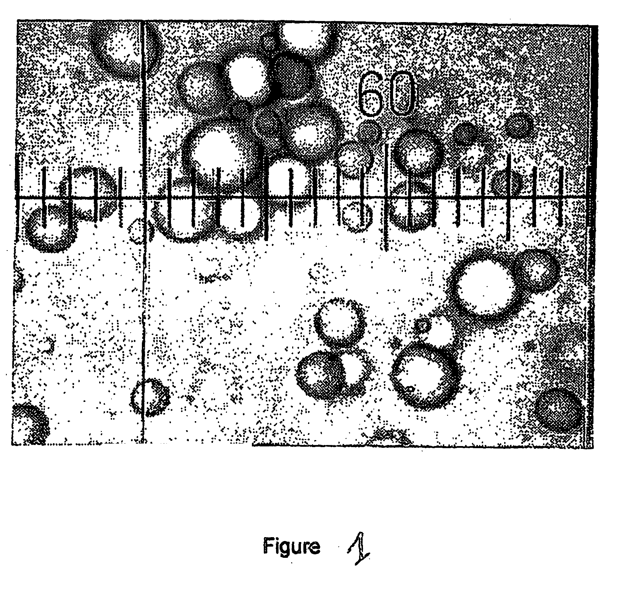

Le 2-éthylhexyl-p-méthoxycinnamate est remplacé dans les exemples 1 à 5 par un mélange de 2-éthyl-p-méthoxycinnamate et 2-éthylhexyl-p-diméthylaminobenzoate.
Dans les divers exemples décrits, les capsules obtenues ont majoritairement des tailles de l'ordre du micromètre ; leur membrane est remarquablement stable aux contraintes physiques de température et au viellissement. Le résidu alkyle sulfaté porté sur la chaîne polymère de la chitine ainsi que la charge cationique (20 mV<potentiel Zeta<50 mV) leur assurent une stabilité bactériologique satisfaisante.
La figure unique annexée est une microscopie de microcapsules de filtre solaire selon l'invention avec une distance entre deux barres verticales de 0,2 μm.

**Revendications**

1. Microcapsules **caractérisées en ce qu'**elles sont formées d'une paroi de chitine ou de dérivés de chitine ou de polyamines polyhydroxylées ou un sel de ceux-ci, ladite paroi enrobant une substance hydrophobe.

2. Microcapsules selon la revendication 1, **caractérisées en ce que** les dérivés de chitine sont choisis parmi les dérivés éthérifiés O-carboxyalkyle, O-hydroxyalkyle, alkyle, les dérivés N-substitués, N-carboxyalkyle, N-hydroxyalkyle, N-alkyle de chitine.

3. Microcapsules selon la revendication 1, **caractérisées en ce que** la paroi de dérivés de chitine ou de polyamines polyhydroxylées résultent de la réticulation du chitosane ou de dérivés de chitosane éthérifiés O-carboxyalkyle, O-hydroxyalkyle, alkyle ou de dérivés de chitosane N-substitués, N-carboxyalkyle, N-hydroxyalkyle, N-alkyle ou de la réticulation ou acétylation de polyamines polyhydroxylées.

4. Microcapsules selon l'une des revendications 1 à 3, **caractérisées en ce que** les sels de chitine ou de dérivés de chitine ou de polyamines polyhydroxylées sont choisis parmi les sels de métal alcalin ou alcalino terreux, d'ammonium, d'acide acétique, d'acide chlorhydrique.

5. Microcapsules selon la revendication 1, **caractérisées en ce que** la substance hydrophobe est choisie parmi les composés à usage cosmétique, pharmaceutique, les agents chromogènes, éventuellement en solution organique.

6. Microcapsules selon la revendication 5, **caractérisées en ce que** la substance hydrophobe est choisie dans le groupe constitué par les filtres solaires.

7. Microcapsules selon la revendication 6, **caractérisées en ce que** les filtres solaires sont choisis dans le groupe suivant :

esters cinnamiques, acide et esters para-amino benzoiques, esters salicyliques, benzylidène camphre et dérivés, benzophénones, dibenzoylméthanes, benzyl diphényl acrylates, anthranilates, triazines, et plus précisemment : octyl méthoxycinnamate, octyl diméthyl PABA, octyl salicylate, homomenthyl salicylate, 4-méthylbenzilidène camphre, benzophénone 3, butyl méthoxy-dibenzoylméthane, octocrylène, menthyl anthranilate, triazone.

8. Microcapsules selon la revendication 7, **caractérisées en ce que** les filtres solaires sont choisis dans le groupe suivant : 2-éthylhexyl-p-méthoxycinnamate, 2-éthylhexyl p-diméthylaminobenzoate, 2-éthylhexyl salicylate, 2-éthylhexyl-2-cyano-3,3-diphényl acrylate, 4-méthylbenzylidène camphre, 2-hydroxy-4-méthoxy benzophénone et 4-4-butyl-4'-méthoxydibenzoylméthane.

9. Microcapsules selon la revendication 1, **caractérisées en ce qu'**elles comprend de 5 à 50 % en poids de substance hydrophobe.

10. Microcapsules selon l'une des revendications précédentes, **caractérisées en ce qu'**elles présentent une taille moyenne comprise entre 0,1 µm et 30 µm.

11. Microcapsules selon la revendication 10, **caractérisées en ce qu'**elles présentent une taille moyenne comprise entre 0,3 µm et 10 µm.

12. Composition à usage cosmétique ou pharmaceutique à usage topique externe comprenant des microcapsules selon l'une des revendications 5 à 11 et un véhicule inerte.

13. Composition selon la revendication 12 à usage topique externe absorbant les UV notamment les UVA ou UVB, comprenant des microcapsules selon l'une des revendications 6 à 11.

14. Composition selon la revendication 12 ou 13, **caractérisée en ce qu'**elle est sous la forme d'un gel, d'un crème, d'une huile.

15. Composition à usage pharmaceutique comprenant des microcapsules contenant une substance pharmaceutique selon la revendication 5 et un véhicule inerte.

16. Procédé de préparation de microcapsules selon l'une des revendications 1 à 11, **caractérisé en ce que** :

    - on forme une émulsion d'une phase hydrophobe constituée de la substance hydrophobe dans une phase aqueuse contenant un agent tensio-actif anionique choisi parmi des tensio-actifs susceptibles de provoquer une insolubilisation de chitosane ou de dérivés de chitosane ou de polyamines polyhydroxylées,
    - on mélange ladite émulsion avec un sel organique de chitosane ou de dérivés de chitosane ou de polyamines polyhydroxylées de façon à provoquer la coacervation du chitosane ou des dérivés de chitosane ou de polyamines polyhydroxylées autour des gouttelettes de phase hydrophobe,
    - on soumet le coacervat de chitosane ou de dérivés de chitosane ou de polyamines polyhydroxylées à une réaction d'acétylation ou de réticulation,
    - on récupère les microcapsules.

17. Procédé de préparation selon la revendication 16, **caractérisé en ce que** l'agent tensio-actif anionique est choisi parmi les alkyles en $C_6$-$C_{18}$, aryle en $C_6$-$C_{10}$, alkyle ($C_1$-$C_{10}$) aryle ($C_6$) ou aryle ($C_6$) alkyle ($C_1$-$C_{10}$) sulfate ou phosphate ou phosphonate dont le contre ion est un métal alcalin ou alcalino terreux.

18. Proeédé de préparation selon la revendication 17, **caractérisé en ce que** le tensio-actif est un alkyle en $C_6$-$C_{18}$, sulfate alcalin, notamment le lauryl sulfate de sodium.

19. Procédé de préparation selon l'une des revendications 16 à 18, **caractérisé en ce que** la phase aqueuse contient un fluide silicone hydrosoluble.

20. Procédé de préparation selon la revendication 16, **caractérisé en ce que** la réaction d'acétylation est effectuée par une solution dans un solvant organique polaire d'un anhydride d'acide organique aliphatique de. 4 à 40 atomes de carbone, aromatique de 12 à 20 atomes de carbone.

**21.** Procédé de préparation selon la revendication 20, **caractérisé en ce que** l'anhydride est choisi dans le groupe constitué par l'anhydride acétique, l'anhydride propionique, l'anhydride butyrique, l'anhydride valérique, l'anhydride benzoïque.

**22.** Procédé de préparation selon la revendication 16, **caractérisé en ce que** la réticulation est effectuée au moyen de glutaraldéhyde ou d'un diisocyanate.

**23.** Procédé de préparation selon la revendication 16, **caractérisé en ce que** le sel de chitosane est l'acétate de chitosane.

**24.** Procédé de préparation selon l'une des revendications précédentes, **caractérisé en ce que** les quantités pondérales des différents réactifs sont les suivantes :

- substance hydrophobe        5 % à 50 %, de préférence 33 à 45 %
- tensio-actifs        0,1 % à 1,5 % de préférence environ 0,33 %
- sel de chitosane ou de dérivés de chitosane ou polyamines
  polyhydroxylées        0,1 % à 10 % de préférence environ 2 %
- anhydride ou        0,1 % à 10 % de préférence environ 0,33 %
- réticulant        0,1 % à 10 % de préférence environ 0,33 %.

**Patentansprüche**

1. Mikrokapseln, **dadurch gekennzeichnet, dass** sie aus einer Wand aus Chitin oder Chitin-Derivaten oder polyhydroxylierten Polyaminen oder einem Salz derselben gebildet sind, wobei die Wand eine hydrophobe Substanz umhüllt.

2. Mikrokapseln nach Anspruch 1, **dadurch gekennzeichnet, dass** die Chitin-Derivate ausgewählt sind aus veretherten O-Carboxyalkyl-, O-Hydroxyalkyl-, Alkyl-Derivaten, aus N-substituierten N-Carboxyalkyl-, N-Hydroxyalkyl-, N-Alkyl-Derivaten von Chitin.

3. Mikrokapseln nach Anspruch 1, **dadurch gekennzeichnet, dass** die Wand aus Chitin-Derivaten oder polyhydroxylierten Polyaminen das Ergebnis der Vernetzung von Chitosan oder veretherten O-Carboxyalkyl-, O-Hydroxyalkyl-, Alkylchitosan-Derivaten oder von N-substituierten N-Carboxyalkyl-, N-Hydroxyalkyl-, N-Alkylchitosan-Derivaten oder der Vernetzung oder Acetylierung von polyhydroxylierten Polyaminen ist.

4. Mikrokapseln nach einem der Ansprüche 1 bis 3, **dadurch gekennzeichnet, dass** die Salze von Chitin oder den Chitin-Derivaten oder polyhydroxylierten Polyaminen ausgewählt sind aus den Alkalimetall- oder Erdalkalimetall-, Ammonium-, essigsauren, Hydrochlorid-Salzen.

5. Mikrokapseln nach Anspruch 1, **dadurch gekennzeichnet, dass** die hydrophobe Substanz ausgewählt ist aus Verbindungen zur kosmetischen, pharmazeutischen Verwendung, farberzeugenden Mitteln, gegebenenfalls in organischer Lösung.

6. Mikrokapseln nach Anspruch 5, **dadurch gekennzeichnet, dass** die hydrophobe Substanz ausgewählt ist aus der Gruppe, die aus den Sonnenfiltern besteht.

7. Mikrokapseln nach Anspruch 6, **dadurch gekennzeichnet, dass** die Sonnenfilter aus der folgenden Gruppe ausgewählt sind:
   Zimtsäureester, para-Aminobenzoesäure und -ester, Salicylsäureester, Benzylidenkampher und Derivate, Benzophenone, Dibenzoylmethane, Benzyldiphenylacrylate, Anthranilate, Triazine und spezieller: Octylmethoxycinnamat, Octyldimethyl-PABS, Octylsalicylat, Homomenthylsalicylat, 4-Methylbenzilidenkampher, Benzophenon 3, Butylmethoxydibenzoylmethan, Octocrylen, Menthylanthranilat, Triazon.

8. Mikrokapseln nach Anspruch 7, **dadurch gekennzeichnet, dass** die Sonnenfilter aus der folgenden Gruppe ausgewählt sind:

2-Ethylhexyl-p-methoxycinnamat, 2-Ethylhexyl-p-dimethylaminobenzoat, 2-Ethylhexylsalicylat, 2-Ethylhexyl-2-cyano-3,3-diphenylacrylat, 4-Methylbenzylidenkampher, 2-Hydroxy-4-methoxybenzophenon und 4,4-Butyl-4'-methoxydibenzoylmethan.

9. Mikrokapseln nach Anspruch 1, **dadurch gekennzeichnet, dass** sie 5 bis 50 Gew.-% an hydrophober Substanz umfassen.

10. Mikrokapseln nach einem der vorangehenden Ansprüche, **dadurch gekennzeichnet, dass** sie eine mittlere Größe zwischen 0,1 μm und 30 um einschließlich aufweisen.

11. Mikrokapseln nach Anspruch 10, **dadurch gekennzeichnet, dass** sie eine mittlere Größe zwischen 0,3 μm und 10 μm einschließlich aufweisen.

12. Zusammensetzung zur kosmetischen oder pharmazeutischen Verwendung für die äußere topische Verwendung, umfassend Mikrokapseln nach einem der Ansprüche 5 bis 11 und ein inertes Vehikel.

13. Zusammensetzung nach Anspruch 12 zur äußeren topischen Verwendung, die UV, insbesondere UVA oder UVB, absorbiert und die Mikrokapseln nach einem der Ansprüche 6 bis 11 umfasst.

14. Zusammensetzung nach Anspruch 12 oder 13, **dadurch gekennzeichnet, dass** sie in Form eines Gels, einer Creme, eines Öls vorliegt.

15. Zusammensetzung zur pharmazeutischen Verwendung, umfassend Mikrokapseln, die eine pharmazeutische Substanz nach Anspruch 5 und ein inertes Vehikel enthält.

16. Verfahren zur Herstellung von Mikrokapseln nach einem der Ansprüche 1 bis 11, **dadurch gekennzeichnet, dass**:

    - man eine Emulsion einer hydrophoben Phase, die aus der hydrophoben Substanz zusammengesetzt ist, in einer wässrigen Phase bildet, welche ein anionisches Grenzflächen-aktives Mittel enthält, das aus Tensiden ausgewählt ist, die geeignet sind, eine Unlöslichmachung von Chitosan oder Chitosan-Derivaten oder polyhydroxylierten Polyaminen hervorzurufen,
    - man die Emulsion mit einem organischen Salz von Chitosan oder Chitosan-Derivaten oder polyhydroxylierten Polyaminen auf eine Weise mischt, dass die Koazervation des Chitosans oder der Chitosan-Derivate oder der polyhydroxylierten Polyamine um die Tröpfchen der hydrophoben Phase herum hervorgerufen wird.
    - man das Koazervat von Chitosan oder Chitosan-Derivaten oder polyhydroxylierten Polyaminen einer Acetylierungs- oder Vernetzungsreaktion unterzieht,
    - man die Mikrokapseln gewinnt.

17. Herstellungsverfahren nach Anspruch 16, **dadurch gekennzeichnet, dass** das anionische Grenzflächen-aktive Mittel ausgewählt ist aus $(C_6-C_{18})$-Alkyl-, $(C_6-C_{10})$-Aryl-, $(C_1-C_{10})$-Alkyl-$(C_6)$-aryl- oder $(C_6)$-Aryl-$(C_1-C_{10})$-alkylsulfat oder -phosphat oder -phosphonat, deren Gegenion ein Alkali-oder Erdalkalimetall ist.

18. Herstellungsverfahren nach Anspruch 17, **dadurch gekennzeichnet, dass** das Tensid ein Alkali-$(C_6-C_{18})$-alkylsulfat, insbesondere Natriumlaurylsulfat, ist.

19. Herstellungsverfahren nach einem der Ansprüche 16 bis 18, **dadurch gekennzeichnet, dass** die wässrige Phase ein wasserlösliches Silikon-Fluid enthält.

20. Herstellungsverfahren nach Anspruch 16, **dadurch gekennzeichnet, dass** die Acetylierungsreaktion mittels einer Lösung eines organischen aliphatischen Säureanhydrids mit 4 bis 40 Kohlenstoffatomen, aromatischen Säureanhydrids mit 12 bis 20 Kohlenstoffatomen, in einem polaren organischen Lösungsmittel bewirkt wird.

21. Herstellungsverfahren nach Anspruch 20, **dadurch gekennzeichnet, dass** das Anhydrid aus der Gruppe ausgewählt ist, die aus Acetanhydrid, Propionsäureanhydrid, Buttersäureanhydrid, Valeriansäureanhydrid, Benzoesäureanhydrid besteht.

22. Herstellungsverfahren nach Anspruch 16, **dadurch gekennzeichnet, dass** die Vernetzung mittels Glutaraldehyd oder eines Diisocyanats bewirkt wird.

23. Herstellungsverfahren nach Anspruch 16, **dadurch gekennzeichnet, dass** das Chitosan-Salz Chitosanacetat ist.

24. Herstellungsverfahren nach einem der vorangehenden Ansprüche, **dadurch gekennzeichnet, dass** die Gewichtsmengen der verschiedenen Reagenzien die folgenden sind:

- hydrophobe Substanz     5 % bis 50 %, vorzugsweise 33 bis 45 %
- Tensid     0,1 % bis 1,5 %, vorzugsweise etwa 0,33 %
- Salz von Chitosan oder Chitosan-Derivaten oder polyhydroxylierten Polyaminen     0,1 % bis 10 %, vorzugsweise etwa 2 %
- Anhydrid oder     0,1 % bis 10 %, vorzugsweise etwa 0,33 %
- Vernetzungsmittel     0,1 % bis 10 %, vorzugsweise etwa 0,33 %.

**Claims**

1. Microcapsules, **characterized in that** they are formed of a wall of chitin or of chitin derivatives or of polyhydroxylated polyamines or a salt of the latter, the said wall enveloping a hydrophobic substance.

2. Microcapsules according to Claim 1, **characterized in that** the chitin derivatives are chosen from O-carboxyalkyl, O-hydroxyalkyl or alkyl etherified derivatives or N-carboxyalkyl, N-hydroxyalkyl or N-alkyl N-substituted derivatives of chitin.

3. Microcapsules according to Claim 1, **characterized in that** the wall of chitin derivatives or of polyhydroxylated polyamines result from the crosslinking of chitosan or of O-carboxyalkyl, O-hydroxyalkyl or alkyl etherified chitosan derivatives or of N-carboxyalkyl, N-hydroxyalkyl or N-alkyl N-substituted chitosan derivatives or from the crosslinking or acetylation of polyhydroxylated polyamines.

4. Microcapsules according to one of Claims 1 to 3, **characterized in that** the salts of chitin or of chitin derivatives or of polyhydroxylated polyamines are chosen from alkali metal or alkaline earth metal, ammonium, acetic acid or hydrochloric acid salts.

5. Microcapsules according to Claim 1, **characterized in that** the hydrophobic substance is chosen from compounds for cosmetic or pharmaceutical use or chromogenic agents, optionally in organic solution.

6. Microcapsules according to Claim 5, **characterized in that** the hydrophobic substance is chosen from the group composed of sunscreens.

7. Microcapsules according to Claim 6, **characterized in that** the sunscreens are chosen from the following group: cinnamic esters, para-aminobenzoic acid and esters, salicylic esters, benzylidenecamphor and derivatives, benzophenones, dibenzoylmethanes, benzyldiphenyl acrylates, anthranilates or triazines and more specifically: octyl methoxycinnamate, octyl dimethyl-PABA, octyl salicylate, homomenthyl salicylate, 4-methylbenzylidenecamphor, 3-benzophenone, butylmethoxydibenzoylmethane, octocrylene, menthyl anthranilate or triazone.

8. Microcapsules according to Claim 7, **characterized in that** the sunscreens are chosen from the following group: 2-ethylhexyl p-methoxycinnamate, 2-ethylhexyl p-dimethylaminobenzoate, 2-ethylhexyl salicylate, 2-ethylhexyl 2-cyano-3,3-diphenylacrylate, 4-methylbenzylidenecamphor, 2-hydroxy-4-methoxybenzophenone and 4,4-butyl-4'-methoxydibenzoylmethane.

9. Microcapsules according to Claim 1, **characterized in that** they comprise from 5 to 50% by weight of hydrophobic substance.

10. Microcapsules according to one of the preceding claims, **characterized in that** they exhibit a mean size of between 0.1 mm and 30 mm.

11. Microcapsules according to Claim 10, **characterized in that** they exhibit a mean size of between 0.3 mm and 10 mm.

12. Composition for cosmetic or pharmaceutical use for external topical use comprising microcapsules according to one of Claims 5 to 11 and an inert vehicle.

13. Composition according to Claim 12 for external topical use which absorbs UV radiation, in particular UVA or UVB radiation, comprising microcapsules according to one of Claims 6 to 11.

14. Composition according to Claim 12 or 13, **characterized in that** it is in the form of a gel, of a cream or of an oil.

15. Composition for pharmaceutical use comprising microcapsules containing a pharmaceutical substance according to Claim 5 and an inert vehicle.

16. Process for the preparation of microcapsules according to one of Claims 1 to 11, **characterized in that**:

   - an emulsion is formed of a hydrophobic phase, composed of the hydrophobic substance, in an aqueous phase containing an anionic surface-active agent chosen from surfactants capable of causing chitosan or chitosan derivatives or polyhydroxylated polyamines to become insoluble,
   - the said emulsion is mixed with an organic salt of chitosan or of chitosan derivatives or of polyhydroxylated polyamines, so as to cause coacervation of the chitosan or of the chitosan derivatives or of the polyhydroxylated polyamines around the droplets of hydrophobic phase,
   - the coacervate of chitosan or of chitosan derivatives or of polyhydroxylated polyamines is subjected to an acetylation or crosslinking reaction,
   - the microcapsules are recovered.

17. Preparation process according to Claim 16, **characterized in that** the anionic surface-active agent is chosen from $C_6$-$C_{18}$ alkyl, $C_6$-$C_{10}$ aryl, $(C_1$-$C_{10})$alkyl$(C_6)$aryl or $(C_6)$aryl$(C_1$-$C_{10})$alkyl sulphate or phosphate or phosphonate, the counterion of which is an alkali metal or alkaline earth metal.

18. Preparation process according to Claim 17, **characterized in that** the surfactant is an alkaline $C_6$-$C_{18}$ alkyl sulphate, in particular sodium lauryl sulphate.

19. Preparation process according to one of Claims 16 to 18, **characterized in that** the aqueous phase contains a water-soluble silicone fluid.

20. Preparation process according to Claim 16, **characterized in that** the acetylation reaction is carried out by a solution, in a polar organic solvent, of an aliphatic organic acid anhydride containing 4 to 40 carbon atoms or an aromatic organic acid anhydride containing 12 to 20 carbon atoms.

21. Preparation process according to Claim 20,
**characterized in that** the anhydride is chosen from the group composed of acetic anhydride, propionic anhydride, butyric anhydride, valeric anhydride and benzoic anhydride.

22. Preparation process according to Claim 16,
**characterized in that** the crosslinking is carried out by means of glutaraldehyde or of a diisocyanate.

23. Preparation process according to Claim 16,
**characterized in that** the chitosan salt is chitosan acetate.

24. Preparation process according to one of the preceding claims, **characterized in that** the amounts by weight of the various reactants are as follows:

   - hydrophobic substance      5% to 50%, preferably 33 to 45%
   - surfactants      0.1% to 1.5%, preferably about 0.33%
   - salt of chitosan or of chitosan derivatives or of polyhydroxylated polyamines      0.1% to 10%, preferably about 2%
   - anhydride      0.1% to 10%, preferably about 0.33% or
   - crosslinking agent      0.1% to 10%, preferably about 0.33%.

Figure 1